# EUROPEAN PATENT APPLICATION

(11) **EP 2 832 351 A1**
(43) Date of publication of application: **04.02.2015**
(21) Application number: 14178449.6
(22) Date of filing: 25.07.2014
(51) Int. Cl.: A61K 9/24, A61K 31/522, A61K 31/675

(54) **Multilayer Tablet Formulations Comprising Tenofovir and Entecavir**

(30) Priority: 29.07.2013 TR 201309159
(71) Applicant: Sanovel Ilac Sanayi ve Ticaret A.S., 34460 Istanbul (TR)
(72) Inventor: Türkyilmaz, Ali, 34460 Istanbul (TR); Yildirim, Ediz, 34460 Istanbul (TR); Erdem, Yelda, 34460 Istanbul (TR)
(74) Representative: Sevinç, Erkan

(57) **Abstract**

The present invention relates to a multilayer tablet formulation comprising tenofovir or a pharmaceutically acceptable salt thereof and entecavir or a pharmaceutically acceptable salt thereof. The present invention further relates to a process for preparing such a formulation and to the use thereof in the treatment of chronic hepatitis B.

## Description

### Field of Invention

The present invention relates to a multilayer tablet formulation comprising tenofovir or a pharmaceutically acceptable salt thereof and entecavir or a pharmaceutically acceptable salt thereof. The present invention further relates to a process for preparing a tablet formulation and to the use thereof in the treatment of chronic hepatitis B.

### Background of Invention

Tenofovir is a nucleotide analogue reverse transcriptase inhibitor. The chemical name of tenofovir is ({[(2R)-1-(6-amino-9H-purin-9-yl)propan-2-yl]oxy}methyl) phosphonic acid and has the structure shown in the following formula I.

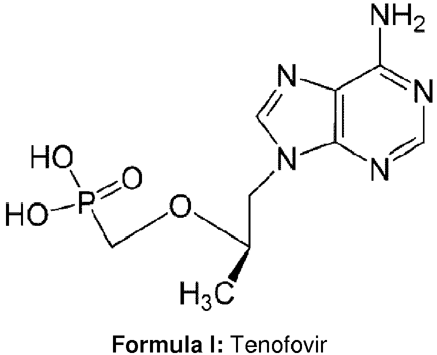

Tenofovir disoproxil fumarate is a prodrug of tenofovir and is marketed under the brand name VIREAD^{®}. Tenofovir disoproxil fumarate blocks the reverse transcriptase enzyme which is crucial for the human immunodeficiency virus 1 (HIV-1) and the hepatitis B virus. Therefore, it is used in the treatment of HIV-1 and hepatitis B. Tenofovir disoproxil fumarate has the following structural formula.

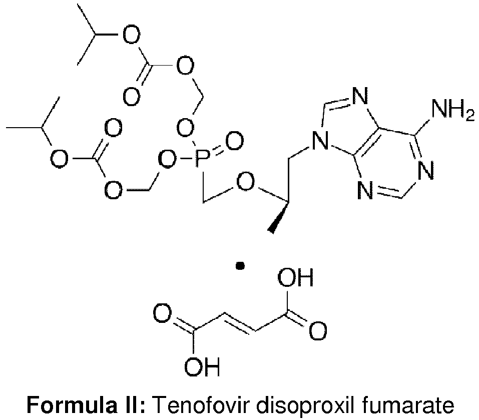

The tenofovir molecule was first disclosed in the patent US5922695A. This patent further claims a method for treating and preventing viral infections and for orally administrating this molecule.

Entecavir is a nucleoside reverse transcriptase inhibitor and is also a guanosine nucleoside analogue showing a selective activity against the hepatitis B virus (HBV). The chemical name of entecavir monohydrate is 2-amino-1,9-dihydro-9-[(1S,3R,4S)-4-hydroxy-3-(hydroxymethyl)-2-methylenecyclopenthyl]-6H-purin-6-one monohydrate. Entecavir monohydrate has the structure shown in the following formula.

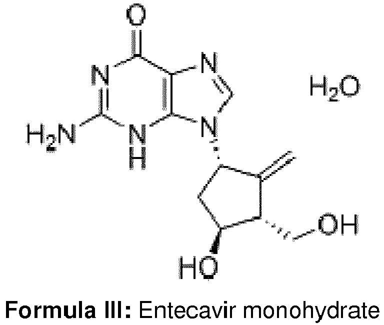

It is marketed under the brand name BARACLUDE^{®} in doses comprising 0.5 mg and 1 mg equivalent of entecavir in the form of oral tablets or oral solutions and is used in the treatment of chronic hepatitis B virus infections in the adults.

The entecavir molecule and the use thereof in the treatment of hepatitis B were disclosed in the patent US5206244. Methods for the synthesis of entecavir were disclosed in the patent WO 98/09964.

On the other hand, a combination of a nucleotide reverse transcriptase inhibitor and a nucleoside reverse transcriptase inhibitor was disclosed in the patent EP2051703B1. In that patent, however, tenofovir and lamuvidine were selected as the nucleotide reverse transcriptase inhibitor and the nucleoside reverse transcriptase inhibitor, respectively. However, since these two inhibitor groups are incompatible, a double-layer tablet form was preferred. On the other hand, an interaction may take place at the contact surface between the layer of the nucleotide reverse transcriptase inhibitor and the layer of the nucleoside reverse transcriptase inhibitor in the double-layer tablet, this interaction deteriorating the stability of the tablet.

It was seen that using more than one antiretroviral drug in the treatment of chronic hepatitis B provided a positive influence on the treatment. Entecavir and tenofovir disoproxil fumarate have an additional or synergistic antiviral activity in the treatment of chronic hepatitis B patients. As stated above, based on the interaction of these two active agents, the most difficult aspect of producing a tablet formulation comprising these two actives is obtaining a tablet which will be stable throughout the shelf life and provide a high bioavailability.

For this reason, there is a need in the relevant field to formulate and to use an oral tablet preparation comprising entecavir monohydrate and tenofovir disoproxil fumarate with a high bioavailability and an improved stability.

### Description of Figures

**Figure 1** illustrates an oral solid multilayer tablet formulation having i) a layer (a) comprising entecavir monohydrate, ii) a layer (b) comprising tenofovir disoproxil fumarate, and iii) an inert layer (c) separating these two layers from each other.

### Detailed Description of Invention

The main object of the present invention is to provide a novel stable multilayer tablet formulation comprising entecavir monohydrate and tenofovir disoproxil fumarate, which overcomes the aforementioned problems according to the prior art and provides additional advantages. Accordingly, a three-layer tablet formulation is developed with an inert layer in the middle. This inert layer eliminates the incompatibility of the two active agents. The layer (a) comprising entecavir monohydrate and the layer (b) comprising tenofovir disoproxil fumarate are immediate-release layers. These two layers are separated using the inert layer (c).

Cellulose derivaties in the inert layer as the intermediate layer of the three-layer tablet formulation comprising entecavir monohydrate and tenofovir disoproxil fumarate surprisingly allowed obtaining a tablet which is stable throughout the shelf life. No speckling or discoloring is observed in the three-layer tablet form comprising these two active agents. The cellulose derivatives in the inert layer are preferably low-substituted hydroxypropyl cellulose and microcrystalline cellulose. Low-substituted hydroxypropyl cellulose can be used as a "dry binder" in tablet formulations comprising a higher amount of microcrystalline cellulose and shows a good compressibility. Additionally, low-substituted hydroxypropyl cellulose in an amount of 1.00 to 15.00% by weight of the inert layer enhances the stability of the tablet and since low-substituted hydroxypropyl cellulose is not ionic, it does not interact with the active agents. It was seen that microcrystalline cellulose, the other cellulose derivative, prevented the interaction between the active agents in the formulation, since it is not dissolvable in water. It was further observed that using microcrystalline cellulose, which known to have a high stability, as filler in an amount of 89.00 to 97.00% by weight of the inert layer contributed increasing the shelf life of the tablet.

Low-substituted hydroxypropyl cellulose is a low-substituted hydroxypropyl ether of cellulose within a small portion of the hydroxypropyl groups in the glucose unit. Whereas hydroxypropylcellulose (Molar Substitution= 3) is soluble in both water and alcohol, low-substituted hydroxypropyl cellulose (Molar Substitution = 0.2-0.4) swells in water and is insoluble.

It was also found with the present invention that setting the ratio of the total weight of low-substituted hydroxypropyl cellulose to the total weight of microcrystalline cellulose in the inert layer between 1:10 and 1:25, preferably between 1:15 and 1:20, increased the compressibility of the intermediate layer and exhibited a synergistic effect on enhancing the stability of the tablet.

According to another embodiment, the ratio of the total weight of entecavir monohydrate to the total weight of tenofovir disoproxil fumarate in the three-layer tablet formulation is between 1:100 and 1:800, preferably between 1:300 and 1:600. Since the percentage of tenofovir disoproxil fumarate is remarkably higher than the percentage of entecavir monohydrate in the tablet formulation, the efficiency of entecavir monohydrate is not reduced due to reasons such as amount losses or lack of providing a uniform distribution by virtue of keeping these two active agents in the different layers of the three-layer tablet. Additionally, uniform layers comprising active agents are obtained and the bioavailability of the tablet is increased by virtue of this three-layer tablet embodiment.

As another embodiment of the present invention, the three-layer tablet formulation comprising entecavir monohydrate and tenofovir disoproxil fumarate includes at least one excipient. As an excipient, the three-layer tablet formulation according to the present invention may comprise lactose monohydrate, microcrystalline cellulose, low-substituted hydroxypropyl cellulose, hydroxypropyl methyl cellulose, mannitol, starch, pregelatinized starch, sodium starch glycolate, croscarmellose sodium, magnesium stearate, colloidal silicon dioxide, sodium stearyl fumarate, colorants, or the mixtures thereof.

As a further embodiment, the present invention provides a three-layer tablet formulation of entecavir monohydrate and tenofovir disoproxil fumarate comprising the following excipients (the percentage amounts of the agents are calculated based on the total weight of the layer in which they are present).

The layer comprising entecavir monohydrate:
a. 0.01 to 1.00% by weight of entecavir monohydrate,
b. 8.00 to 12.00% by weight of starch,
c. 8.00 to 12.00% by weight of sodium starch glycolate,
d. 45.00 to 50.00% by weight of microcrystalline cellulose,
e. 22.00 to 28.00% by weight of mannitol,
f. 1.00 to 5.00% by weight of hydroxypropyl methyl cellulose,
g. 1.00 to 5.00% by weight of sodium stearyl fumarate,
h. 0.01 to 2.00% by weight of colloidal silicon dioxide;

The inert layer:
a. 1.00 to 15.00% by weight of low-substituted hydroxypropyl cellulose,
b. 89.00 to 97.00% by weight of microcrystalline cellulose,
c. 0.01 to 2.00% by weight of colloidal silicon dioxide,
d. 0.01 to 2.00% by weight of yellow iron oxide,
e. 0.10 to 3.00% by weight of magnesium stearate;

The layer comprising tenofovir disoproxil fumarate:
a. 40.00 to 50.00% by weight of tenofovir disoproxil fumarate,
b. 18.00 to 25.00% by weight of lactose monohydrate,
c. 7.00 to 13.00% by weight of pregelatinized starch,
d. 12.00 to 18.00% by weight of microcrystalline cellulose,
e. 3.00 to 9.00% by weight of croscarmellose sodium,
f. 0.10 to 3.00% by weight of magnesium stearate.

As another embodiment, the present invention provides a process for the preparation of a three-layer tablet formulation of entecavir monohydrate and tenofovir disoproxil fumarate comprising the following steps.
→ The layer comprising entecavir monohydrate:
a. entecavir monohydrate, starch, 20% of mannitol, 25% of microcrystalline cellulose, and hydroxypropyl methyl cellulose are sieved and taken to a granulator,
b. the resulting powder mixture is granulated with a hydrochloride solution,
c. the resulting granules are dried to a desired moisture content in an oven,
d. the dried granules are passed through a sieve, the remaining amount of microcrystalline cellulose, the remaining amount of mannitol, and sodium starch glycolate are added therein and mixed together, and
e. sodium stearyl fumarate and colloidal silicon dioxide are added therein and stirred until a homogeneous mixture is obtained;
→ the inert layer:
a. microcrystalline cellulose, low-substituted hydroxypropyl cellulose, yellow iron oxide, colloidal silicon dioxide, and magnesium stearate are mixed until a homogeneous mixture is formed;
→ the layer comprising tenofovir disoproxil fumarate:
a. tenofovir disoproxil fumarate, pregelatinized starch, and lactose monohydrate are taken to a granulator and mixed therein,
b. the resulting powder mixture is granulated with water,
c. the resulting granules are sieved and then dried to a desired moisture content in an oven,
d. microcrystalline cellulose and croscarmellose sodium are added to the dry granules and mixed together,
e. magnesium stearate is added and the resulting mixture is stirred until a homogeneous mixture is formed;
→ Those three different mixtures obtained are pressed in the form of three-layer tablets so that the inert layer is formed in the middle, i.e. forms the intermediate layer.

The present invention is described in the following examples in more details. These examples are not limiting the scope of the present invention and should be considered under the light of the foregoing detailed disclosure.

### Example: Three-layer tablet comprising entecavir monohydrate and tenofovir disoproxil fumarate

| ***1^{st} Layer*** | ***Amount (%)*** |
|---|---|
| Entecavir monohydrate | 0.01 - 1.00 |
| Starch | 8.00 - 12.00 |
| Sodium starch glycolate | 8.00 - 12.00 |
| Microcrystalline cellulose | 45.00 - 50.00 |
| Mannitol | 22.00 - 28.00 |
| Hydroxypropyl methylcellulose | 1.00 - 5.00 |
| Sodium stearyl fumarat | 1.00 - 5.00 |
| Colloidal silicon dioxide | 0.01 - 2.00 |

| ***Inert layer*** | ***Amount (%)*** |
|---|---|
| Low-substituted hydroxypropyl cellulose | 1.00 - 15.00 |
| Microcrystalline cellulose | 89.00 - 97.00 |
| Colloidal silicon dioxide | 89.00 - 97.00 |
| Yellow iron oxide | 0.01 - 2.00 |
| Magnesium stearate | 0.10 - 3.00 |

| ***3^{rd} Layer*** | ***Amount (%)*** |
|---|---|
| Tenofovir disoproxil fumarate | 40.00 - 50.00 |
| Lactose monohydrate | 18.00 - 25.00 |
| Pregelatinized starch | 7.00 - 13.00 |
| Microcrystalline cellulose | 12.00 - 18.00 |
| Croscarmellose sodium | 3.00 - 9.00 |
| Magnesium stearate | 0.10 - 3.00 |

| | |
|---|---|
| * *The percentages given for the agents are calculated based on the total weight of the respective layer.* | |

First layer:
- entecavir monohydrate, starch, 20% of mannitol, 25% of microcrystalline cellulose, and hydroxypropyl methyl cellulose are sieved and taken to a granulator,
- the resulting powder mixture is granulated with a hydrochloride solution,
- the resulting granules are dried to a desired moisture content in an oven,
- the dried granules are passed through a sieve, the remaining amount of microcrystalline cellulose, the remaining amount of mannitol, and sodium starch glycolate are added therein, and
- sodium stearyl fumarate and colloidal silicon dioxide are added therein and stirred until a homogeneous mixture is formed;

Inert layer:
- microcrystalline cellulose, low-substituted hydroxypropyl cellulose, yellow iron oxide, colloidal silicon dioxide, and magnesium stearate are stirred until a homogeneous mixture is formed;

Third layer:
- tenofovir disoproxil fumarate, pregelatinized starch, and lactose monohydrate are taken to a granulator and mixed therein,
- the resulting powder mixture is granulated with water,
- the resulting granules are dried to a desired moisture content in an oven,
- microcrystalline cellulose and croscarmellose sodium are added to the dry granules, and
- magnesium stearate is added and the resulting mixture is stirred until a homogeneous mixture is formed.

These three different mixtures obtained are pressed in the form of three-layer tablets so that the inert layer is formed in the middle, i.e. forms the intermediate layer.

## Claims

1. A three-layer oral tablet formulation comprising entecavir or a pharmaceutically acceptable salt thereof and tenofovir or a pharmaceutically acceptable salt thereof.

2. The three-layer tablet formulation according to claim 1, comprising an immediate release layer comprising entecavir monohydrate, an immediate release layer comprising tenofovir disoproxil fumarate, and an inert layer separating these two layers from each other.

3. The three-layer tablet formulation according to claims 1 and 2, wherein the inert layer comprising cellulose derivatives.

4. The three-layer tablet formulation according to claim 3, wherein the cellulose derivatives in the inert layer are low-substituted hydroxypropyl cellulose and microcrystalline cellulose.

5. The three-layer tablet formulation according to claim 4, wherein the amount of low-substituted hydroxypropyl cellulose is in the range of 1.00 to 15.00% by weight of the inert layer.

6. The three-layer tablet formulation according to claim 4, wherein the amount of microcrystalline cellulose is in the range of 89.00 to 97.00% by weight of the inert layer.

7. The three-layer tablet formulation according to claims 3 to 6, wherein the ratio of the weight of low-substituted hydroxypropyl cellulose to the weight of microcrystalline cellulose in the inert layer is between 1:10 and 1:25.

8. The three-layer tablet formulation according to claims 3 to 6, wherein the ratio of the weight of low-substituted hydroxypropyl cellulose to the weight of microcrystalline cellulose in the inert layer is between 1:15 and 1:20.

9. The three-layer tablet formulation according to claims 1 and 2, wherein the ratio of the total weight of entecavir monohydrate to the total weight of tenofovir disoproxil fumarate is between 1:100 and 1:800 in the three-layer tablet formulation.

10. The three-layer tablet formulation according to claim 9, wherein the ratio of the total weight of entecavir monohydrate to the total weight of tenofovir disoproxil fumarate is between 1:300 and 1:600 in the three-layer tablet formulation.

11. The three-layer tablet formulation according to any of the preceding claims, wherein each layer comprising at least one excipient.

12. The three-layer tablet formulation according to claim 11, wherein the excipient is selected from lactose monohydrate, microcrystalline cellulose, low-substituted hydroxypropyl cellulose, hydroxypropyl methyl cellulose, mannitol, starch, pregelatinized starch, sodium starch glycolate, croscarmellose sodium, magnesium stearate, colloidal silicon dioxide, sodium stearyl fumarate, colorants, or the mixtures thereof.

13. The three-layer tablet formulation according to any of the preceding claims, comprising the following agents, wherein the percentage amounts of the agents are calculated based on the total weight of the layer in which they are present,
the layer comprising entecavir monohydrate:
a) 0.01 to 1.00% by weight of entecavir monohydrate,
b) 8.00 to 12.00% by weight of starch,
c) 8.00 to 12.00% by weight of sodium starch glycolate,
d) 45.00 to 50.00% by weight of microcrystalline cellulose,
e) 22.00 to 28.00% by weight of mannitol,
f) 1.00 to 5.00% by weight of hydroxypropyl methyl cellulose,
g) 1.00 to 5.00% by weight of sodium stearyl fumarate,
h) 0.01 to 2.00% by weight of colloidal silicon dioxide;
the inert layer:
a) 1.00 to 15.00% by weight of low-substituted hydroxypropyl cellulose,
b) 89.00 to 97.00% by weight of microcrystalline cellulose,
c) 0.01 to 2.00% by weight of colloidal silicon dioxide,
d) 0.01 to 2.00% by weight of yellow iron oxide,
e) 0.10 to 3.00% by weight of magnesium stearate;
the layer comprising tenofovir disoproxil fumarate:
a) 40.00 to 50.00% by weight of tenofovir disoproxil fumarate,
b) 18.00 to 25.00% by weight of lactose monohydrate,
c) 7.00 to 13.00% by weight of pregelatinized starch,
d) 12.00 to 18.00% by weight of microcrystalline cellulose,
e) 3.00 to 9.00% by weight of croscarmellose sodium,
f) 0.10 to 3.00% by weight of magnesium stearate.

14. The process for preparing a three-layer tablet formulation according to any of the preceding claims, comprising the following steps,
for the layer comprising entecavir monohydrate:
a) sieving entecavir monohydrate, starch, 20% of mannitol, 25% of microcrystalline cellulose, and hydroxypropyl methyl cellulose and taking these ingredients into a granulator,
b) granulating the resulting powder mixture with a hydrochloride solution,
c) drying the resulting granules to a desired moisture content in an oven,
d) passing the dried granules through a sieve, and adding the remaining amount of microcrystalline cellulose, the remaining amount of mannitol, and sodium starch glycolate therein, and
e) adding sodium stearyl fumarate and colloidal silicon dioxide therein and stirring this mixture until a homogeneous mixture is obtained;
for the inert layer:
a) mixing microcrystalline cellulose, low-substituted hydroxypropyl cellulose, yellow iron oxide, colloidal silicon dioxide, and magnesium stearate until a homogeneous mixture is formed;
for the layer comprising tenofovir disoproxil fumarate:
a) taking tenofovir disoproxil fumarate, pregelatinized starch, and lactose monohydrate into a granulator and mixing these ingredients therein,
b) granulating the resulting powder mixture with water,
c) sieving the resulting granules and then drying the granules to a desired moisture content in an oven,
d) adding microcrystalline cellulose and croscarmellose sodium to the dry granules, and
e) adding magnesium stearate and mixing the resulting mixture until a homogeneous mixture is formed;
wherein these three different mixtures obtained are pressed in the form of three-layer tablets so that the inert layer is formed in the middle, i.e. forms the intermediate layer.

15. The pharmaceutical formulation according to any of the preceding claims for use in the prevention or treatment of the chronic hepatitis B diseases.
